# EUROPEAN PATENT APPLICATION

(11) **EP 3 862 020 A1**
(43) Date of publication of application: **11.08.2021**
(21) Application number: 20202228.1
(22) Date of filing: 16.10.2020
(51) Int. Cl.: A61K 47/00, A61K 9/00, A61K 8/34, A61K 8/00

(54) **RAPID SET GEL CONCENTRATE FOR MEDICAL PRODUCTS**

(30) Priority: 07.02.2020 US 202062995669 P; 27.03.2020 US 202016832461
(71) Applicant: Fenzl, Mark Edward, Sarasota, FL 34233 (US)
(72) Inventor: Fenzl, Mark Edward, Sarasota, FL 34233 (US)
(74) Representative: Sach, Greg Robert

(57) **Abstract**

A concentrate for preparation of a gel for one of a medical device, a medical product, or a first aid product includes at least one gelling agent, at least one humectant, and at least one preservative. A gel is prepared from water and the. A method of preparing the gel includes the step of admixing water and the concentrate. The concentrate of the present disclosure is configured to be added to be sold, transported, and stored in concentrated form to ease transportation, decrease the cost of transportation and storage, optimize storage space, minimize product cost, ease use of product, militate against risk of microbial contamination, and decrease risk of product breakdown with exposure to UV light.

## Description

### FIELD

The present disclosure relates to concentrates and, more particularly, to a concentrate for preparation of medical products such as ultrasound gel.

### BACKGROUND

Ultrasonography is the process of using an ultrasound machine that transmits ultrasound waves for a diagnostic or therapeutic purpose. Ultrasonography requires ultrasound gel to transmit ultrasound waves to and from the transducer on the ultrasound machine to the patient and back. The process requires the application of ultrasound gel or lotion to the patient.

There are several problems with current ultrasound gels. Ultrasound gel comes in 250 ml bottles and 5 liter containers. The 250 ml bottles are relatively expensive and, when discarded, cause excess waste. The 5 liter containers are opened multiple times over a significant period of time, increasing the risk of contamination by pathologic microbes in a hospital. This can lead to cross-contamination and illness in the hospital. It is also difficult and time consuming for hospital staff to pour the thick gel into smaller containers. Shipping is also a problem. It is expensive to ship ultrasound gel and wasteful to dispose of the cardboard containers.

Ultrasound gels often come in transparent or translucent containers. This may expose the ultrasound gel to ultraviolet light, which may cause breakdown of the chemicals and compromise the safety of the ultrasound gel. Concentrated gels are less translucent and would be less susceptible to ultraviolet damage.

There is a continuing need for a concentrate for preparation of a medical device, medical product, or first aid product, which is configured to be mixed on site. Desirably, the concentrate is resistant to microbial growth.

### SUMMARY

In concordance with the instant disclosure, a concentrate for preparation of a medical device, medical product, or first aid product, which is configured to be mixed onsite and is resistant to microbial growth, has surprisingly been discovered.

The present application relates to a gel adapted to be manufactured, transported, and sold in a concentrated form that can only be used after water or an alcohol is added.

In one embodiment, a concentrate for preparation of an ultrasound gel, includes
at least one gelling agent, at least one humectant, and at least one preservative.

In another embodiment, an ultrasound gel is prepared from water and a concentrate for preparation of an ultrasound gel, including at least one gelling agent, at least one humectant, and at least one preservative.

In a further embodiment, a method of preparing a hand sanitizer includes a step of admixing alcohol and the concentrate for preparation of an hand sanitizer. The hand sanitizer includes at least one gelling agent, at least one humectant, and at least one preservative.

The concentrate of the present disclosure is configured to be sold, transported, and stored in a concentrated form to ease transportation. In addition, the concentrate of the present disclosure will decrease the cost of transportation and storage; optimize storage space; be cost-effective; be easier to use; militate against the risk of microbial contamination; and decrease the risk of product breakdown with exposure to UV light.

Currently medical devices, medical products, or first aid products are not transported or sold in a concentrated form. It is manufactured, transported, and sold in a gel form. There are several benefits of a concentrated medical device, medical product, or first aid product. The first is easier and more cost-effective shipping. A certain percentage of medical devices, medical products, or first aid products are water that increases weight and space during shipping Shipping size may also be smaller as the packaging for shipping could also be much smaller.

A second benefit of concentrated medical devices, medical products, or first aid products is that it they takes up less space than regular medical devices, medical products, or first aid products in storage. This benefits health care facilities by decreasing the amount of required storage space.

Ease of accessibility and cost is also a benefit. Instead of having to try to pour more expensive medical devices, medical products, or first aid products out of a large container into a smaller container, one just has to squeeze a less expensive concentrated medical device, medical product, or first aid product into a water bottle of purified water that is available at local stores.

Concentrated medical device, medical product, or first aid product has a lower water activity level, or amount of water available for microbes to use. This minimizes the likelihood of microbial contamination prior to use and increases safety of the product. Bacteria and candida cannot multiply in a product with a water activity level less than 0.87.

In most particular embodiments, a rapid set gel concentrate for medical devices contains at least one gelling agent, at least one humectant, and at least one preservative. The rapid set gel concentrate can be admixed with other ingredients to create the final product or can be sold as the final product. The final product has a water activity level of less than 0.87 and requires the addition of water prior to use. The rapid set gel concentrate may be used in at least one of an ultrasound conducting product, a sterile ultrasound gel, provided as a packet or capsule, provided as a disintegrating packet, provided in an all-natural product as defined by FDA guidelines, an electrically conductive gel, and a disinfectant, as non-limiting examples.

In yet another particular embodiment, a rapid set gel concentrate for medical supplies or first aid supplies contains at least one gelling agent, at least one humectant, and at least one preservative. The rapid set gel concentrate can be admixed with other ingredients to create the final product or can be sold as the final product. The final product has a water activity level of less than 0.87 and requires the addition of water prior to use. The rapid set gel concentrate may be used in a water soluble lubricant, a sterile water soluble lubricant, a sanitizer, a wound gel, a burn gel, a product containing aloe vera powder, an ulcer gel, a massage gel, a sunscreen, and an insect repellant, as non-limiting examples.

In yet a further particular embodiment, a rapid set gel concentrate for alcohol sanitizers contains at least one gelling agent and at least one humectant. The rapid set gel concentrate can be admixed with other ingredients to create the final product or can be sold as the final product. The final product would have a water activity level of less than 0.87 and would require the addition of an alcohol prior to use.

### DETAILED DESCRIPTION

The following description is merely exemplary in nature and is not intended to limit the present disclosure, application, or uses. In respect of the methods disclosed, the order of the steps presented is exemplary in nature, and thus, is not necessary or critical unless otherwise disclosed.

The terminology used in the specification provided herein below is hereby defined to include similar and/or equivalent terms, and/or alternative embodiments that would be considered obvious to one skilled in the art given the teachings of the present patent application. Additionally, the words "a," "an," and "one" are defined to include one or more of the referenced item unless specifically stated otherwise. Also, the terms "have," "include," "contain," and similar terms are defined to mean "comprising" unless specifically stated otherwise.

Except where otherwise expressly indicated, all numerical quantities in this description are to be understood as modified by the word "about" in describing the broadest scope of the technology.

The term "ultrasound gel" refers to scanning gel or gels, transmission gel or gels, ultrasound gel or gels, ultrasound lotion or lotions, or any other solution designed or sold for the purpose of transmitting ultrasound waves.

The term "preservative" refers to a substance used to militate against decay.

The use of percentages in describing particular formulations hereinbelow should be understood as referring to percentage by weight relative to a total weight of the formulation, unless otherwise disclosed.

The present disclosure is directed to a concentrate for the preparation of an ultrasound gel. The concentrate may include at least one gelling agent, at least one humectant, and at least one preservative.

The gelling agent may be configured to provide a viscosity that is suitable for use as ultrasound gel. Also, the gelling agent may provide low irritancy and non-sensitizing properties. In addition, the gelling agent may have properties such that the gelling agent is not bio-absorbed or metabolized where utilized in the ultrasound gel. The gelling agent may further be dispersed in water without pre-dispersion. In some embodiments, the gelling agent may have a moisture level less than about 10%.

The gelling agent may have a pH in water which is suitable for use on human skin. More particularly, the pH of the gelling agent may range from about 6.0 to about 8.0, more particularly, the pH may be from about 6.0 to about 7.5. In certain embodiments, the gelling agent may be "pre-neutralized," such that no additional ingredients are required to adjust the pH of the gelling agent. In further embodiments, the gelling agent may include a basic additive, which may increase the overall pH of the ultrasound gel in order to reach the suitable pH range disclosed herein.

In a non-limiting example, the gelling agent may be sodium carbomer. Sodium carbomer may be present in the concentrate at a range from about 1 gram to about 10 grams, more particularly, from about 3 grams to about 7 grams, and most particularly, 5 grams. The concentration of sodium carbomer in the ultrasound gel may be from about 0.2% to about 1.5%. A skilled artisan may select other suitable amounts of sodium carbomer for both the concentrate and the final ultrasound gel, as desired.

In a particular example, the sodium carbomer may be a preneutralized carbomer commercially available from the Lotioncrafter® company in Eastsound, Washington. The preneutralized carbomer is a white hygroscopic powder with a moisture of less than 10 percent, and a Brookfield viscosity (25°C, 0.5% in H2O, cps (spindle 7, 20 rpm)) of between 35, 000 and 55,000.

In another example, the sodium carbomer employed as the gelling agent may include Carbomer 940. Carbomer 940 is a fine white powder of a polyvinyl carboxy polymer. Carbomer 940 is cross linked with ethers of pentaerythritol. Carbomer 940 imparts a softness and glide, comparable to glycols, when used to form the gel in accordance with the present disclosure.

Although the above-mentioned carbomers has been found to be useful, one of ordinary skill in the are may also employ other suitable gelling agents within the scope of the disclosure.

In further embodiments, the concentrate may include natural ingredients. In these embodiments, the gelling agent may be vegetable glycerin, gum tragacanth, konjac gum, xanthan gum, or a combination thereof. A skilled artisan may select other suitable gelling agents, as desired.

Water activity may be defined as the ratio of the vapor pressure of water in a substance to the vapor pressure of pure water at the same temperature. Microorganisms require a minimum level of water activity in order to grow and, thus, water activity reduction may be used to control microbial growth.

Accordingly, it is desirable for the concentrate to have a low water activity level. For example, the water activity level of the concentrate may be less than about 0.87. The at least one humectant may be used to reduce the water activity level of the concentrate. The humectant may bind to the free water molecules in the concentrate and thereby reduce the water activity level. As non-limiting examples, the humectant may be nitric acid, dextrose, fructose, glycerol, glycine, vegetable glycerine, glucose, honey, malic acid, salt, sorbitol, sucrose, and tartaric acid.

In one non-limiting example, the humectant may be glycerol. Advantageously, glycerol may militate against undesirable clumping within the concentrate, and therefore, improve the dispersibility of the concentrate. Glycerol may be present in the concentrate at a range from about 0 grams to about 100 grams, more particularly, from about 25 grams to about 75 grams, and most particularly, about 50 grams. A skilled artisan may select other suitable humectants and amounts of humectant in the concentrate, as desired.

The at least one preservative may be non-irritating and non-sensitizing. The preservative may be non-reactive such that the preservative does not undesirably react with other components of the concentrate, the air, or light. The at least one preservative may be effective against gram-negative and gram-positive bacteria, and the yeast Candida albicans, for example. Desirably, the preservative may be not release formaldehyde and may be paraben free. The at least one preservative may also be a combination of multiple preservatives. As non-limiting examples, the preservative may be one of citric acid, potassium sorbate, sorbic acid, and phytocide black currant powder.

In one particular embodiment, the preservative may be phenoxyethanol. Phenoxyethanol may be present in the concentrate at a range from about 0 grams to about 20 grams, more particularly, from about 5 grams to about 15 grams, and most particularly, about 10 grams. A skilled artisan may select other suitable preservatives and amounts of humectant in the concentrate, as desired.

In certain embodiments, the concentrate may include at least one UV blocker. The UV blocker may be one or more chemicals used to block ultraviolet light. The UV blocker may absorb or reflect UV light. Advantageously, the UV blocker may militate against the concentrate coming into contact with UV light, which may militate against premature degradation (colors fading, scents decomposing, etc.) of the concentrate. The UV blocker may be selected from a list of approved UV blocking ingredients including aminobenzoic acid, avobenzone, cinoxate, oxybenzone, homosalate, meradimate, octocrylene, octinoxate, octisalate, oxybenzone, Padimate O, ensulizole, sulisobenzone, titanium dioxide, trolamine salicylate, and zinc oxide. In further embodiments, the concentrate may be provided in a package that blocks UV light. As non-limiting examples, the concentrate may be provided in a light proof bottle or individual UV blocking packets.

In a most particular non-limiting example, an ultrasound gel concentrate may comprise about 5% vegetable glycerine, about 0.5 % Sodium carbomer, about 0.7% phenolxyethanol, and about 0.1 % sorbic acid, with the percentages being weight percentages based on a final gel product after water is added. This solution is admixed and is placed in a disintegrating packet and sealed. When ultrasound gel is needed, the packet is placed into an 8 ounce bottle containing 6 ounces of water. The bottle is closed, the contents are shaken, and after a short period of time an ultrasound gel is available for use.

It should be appreciated that the concentrate may be provided in a plurality of forms. In one embodiment, the concentrate may be provided as a concentrated liquid configured to be admixed and thereby diluted with water to form the ultrasound gel. In other embodiments, the concentrate may be a power configured to be admixed and dissolved in water to form the ultrasound gel.

As disclosed herein, the concentrate may be provided in various final packaging. In certain embodiments, the packaging may be UV light blocking. Where the concentrate is in a liquid form, the concentrate may be provided in a bottle. The bottle may be used to dispense a predetermined amount of the concentrate, as needed.

Where the concentrate is in a powdered form, the powder may be provided in bulk. A user may measure a predetermine amount of the powder, as needed. Alternatively, the powder may be pressed into tablets. The tablet may contain the predetermined amount of powder, such that a user does not need to measure the powder.

In other embodiments, the concentrate may be provided in a dissolvable packet. The dissolvable packet may contain the concentrate in powder form or in liquid form. The user may select one dissolvable packet, as needed, and place the packet in water, where the packet will dissolve, and the concentrate may admix with water to form the ultrasound gel. As one non-limiting example, the dissolvable packet may be thermoformed. Sheets of water-soluble film may be sealed together to encapsulate the concentrate. For example, the sheets may be fabricated from polyvinyl alcohol, which is a water-soluble synthetic polymer. The sheets may be heat sealed, as a non-limiting example. A skilled artisan may select other suitable methods and materials to encapsulate the concentrate, as desired.

The present disclosure further contemplates a method of preparing the ultrasound gel. The method includes a first step of providing the concentrate. As disclosed hereinabove, the concentrate may be provided in a plurality of forms and packaging.

The method may then include a second step of admixing water and the concentrate. More particularly, where the user requires the ultrasound gel, the user may transfer the predetermined amount of the concentrate, for example, a single dissolvable packet or a single tablet, to a bottle configured for the final use. The user may then place a predetermined amount of water to the bottle. The user may then mix the concentrate and water until the ultrasound gel is formed. Optionally, the user may adjust the viscosity by adding more water, as desired.

While the concentrate has been described for use in forming the ultrasound gel, it should be appreciated that the present disclosure contemplates a variety of final uses for the gel formed from the concentrate where admixed with water. As non-limiting examples, end uses for the gel may include lubricants, sanitizers, wound gels, burn gels, ulcer gels, massage gels, sunscreens, and insect repellants. A skilled artisan may utilize the concentrate in suitable uses, which require a gel.

It should be appreciated that further ingredients may be admixed into the final gel, as needed, based on the final use. As one non-limiting example, alcohol may be admixed with the concentrate and water to form a sanitizing gel.

In yet another embodiment of the disclosure, an alcohol such as ethanol may be used instead of water to provide sanitization effect, for example, where the concentrate is adapted for use as a hand sanitizer.

Other ingredients can be included in the present compositions, such as various excipients, including one or more antiadherents (e.g., magnesium stearate), binders (e.g., saccharides, gelatin, polymers), disintegrants (e.g., polyvinylpyrrolidone, carboxymethyl cellulose, modified starches), scents, and colors. A skilled artisan may select suitable excipients in order to facilitate both the concentration of the ingredients into a container, dissolvable packet, or table, and the later preparation of the final gel products, as desired.

As may be presented herein, the language "consisting essentially of" is meant to limit the scope of the claim to the specified materials that do not materially affect the basic and novel characteristics of the additive. It should be appreciated that ingredients that materially affect the additive may adversely affect the water activity level or the gelling properties of the concentrate. Thus, the concentrate consisting essentially of certain ingredients described hereinabove, excludes ingredients that may improve or adversely affect the water activity level or the gelling properties of the concentrate, which would materially affect the basic and novel characteristics of the concentrate.

Additionally, as may be presented in the claims below, the language "consisting of" is intended to exclude any ingredient not specified in the claim. Accordingly, the additive consisting of certain ingredients described hereinabove includes only those ingredients.

While certain representative embodiments and details have been shown for purposes of illustrating the invention, it will be apparent to those skilled in the art that various changes may be made without departing from the scope of the disclosure, which is further described in the following appended claims.

## Claims

1. A concentrate for preparation of a medical device, medical product, or first aid product, comprising:
at least one gelling agent;
at least one humectant; and
at least one preservative.

2. The concentrate of Claim 1, wherein the at least one gelling agent is sodium carbomer.

3. The concentrate of Claim 1, wherein the at least one humectant is glycerol.

4. The concentrate of Claim 1, wherein the at least one preservative is phenoxyethanol.

5. The concentrate of Claim 1, wherein the concentrate is a concentrated liquid.

6. The concentrate of Claim 1, wherein the concentrate is in a water-soluble capsule.

7. The concentrate of Claim 1, wherein the concentrate is a powder.

8. The concentrate of Claim 7, wherein the powder is pressed into a tablet.

9. The concentrate of Claim 1, wherein the concentrate has a water activity level of less than about 0.87.

10. A gel for one of a medical device, medical product, and first aid product, comprising:
a concentrate having at least one gelling agent, at least one humectant, and at least one preservative; and an added solution of at least one of water and an alcohol.

11. The gel of Claim 10, wherein the gel is for a sanitizer.

12. The concentrate of Claim 10, wherein the concentrate has a water activity level of less than about 0.87.

13. A method of preparing a gel for one of a medical device, a medical product, and a first aid product, the method comprising the steps of:
admixing at least one of water and an alcohol with a concentrate, the concentrate having at least one gelling agent, and at least one humectant, and the concentrate further having at least one preservative when water is admixed with the concentrate; whereby the gel is formed.

14. The method of Claim 13, wherein the gel is for a sanitizer.

## Amended claims

### Amended claims in accordance with Rule 137(2) EPC.

**1.** A concentrate suitable for preparation of a medical device, medical product, or first aid product, comprising:
at least one gelling agent, which is an additive used to thicken and stabilize the said concentrate;
at least one humectant; which is an additive used to reduce the loss of moisture from the said concentrate;
at least one preservative; which is an additive used to preserve the said concentrate against microbial attack;
wherein the concentrate has a water activity level of less than about 0.87 which means the vapor pressure of the said concentrate is 87 percent of that of pure water.

**2.** The concentrate of Claim 1, wherein the at least one gelling agent is sodium carbomer.

**3.** The concentrate of Claim 1, wherein the at least one humectant is glycerol.

**4.** The concentrate of Claim 1, wherein the at least one preservative is phenoxyethanol.

**5.** The concentrate of Claim 1, wherein the concentrate is a concentrated liquid or powder.

**9.** The concentrate of Claim 1, wherein the concentrate has a water activity level of less than about 0.87.

**10.** A process of making a conducive medium gel for medical devices , a medical product, and a first aid product, comprising:
a concentrate having at least one gelling agent at a range of 3 grams to about 7 grams; at least one humectant at a range of 25 grams to about 75 grams; and at least one preservative at a range from about 5 grams to about 15 grams; and an added solution of at least one of water and an alcohol.

**12.** The concentrate of Claim 10, wherein the concentrate has a water activity level of less than about 0.87.

**13.** A method of preparing a gel for one of a medical device, a medical product, and a first aid product according to claim 10, the method comprising the steps of:
admixing at least one of water and an alcohol with a concentrate, the concentrate having at least one gelling agent at a range of 3 grams to about 7 grams; and at least one humectant at a range of 25 grams to about 75 grams; and the concentrate further having at least one preservative at a range from about 5 grams to about 15 grams; when water is admixed with the concentrate; whereby the gel is formed.
